(19) Europäisches Patentamt / European Patent Office / Office européen des brevets

(11) **EP 4 135 613 B1**

(12) **EUROPEAN PATENT SPECIFICATION**

(45) Date of publication and mention
of the grant of the patent:
**18.06.2025 Bulletin 2025/25**

(21) Application number: **21716790.7**

(22) Date of filing: **13.04.2021**

(51) International Patent Classification (IPC):
*A61B 34/10* (2016.01)     *A61B 34/20* (2016.01)
*A61B 18/02* (2006.01)     *A61B 17/32* (2006.01)
*A61B 18/18* (2006.01)     *A61B 18/20* (2006.01)
*A61B 18/14* (2006.01)

(52) Cooperative Patent Classification (CPC):
**A61B 34/10;** A61B 18/148; A61B 2017/320069;
A61B 2018/00547; A61B 2018/00577;
A61B 2018/00613; A61B 2018/0212;
A61B 2018/1861; A61B 2018/2005; A61B 2034/107;
A61B 2034/2051; A61B 2034/252; A61B 2034/254;
A61B 2090/368; A61B 2090/374;          (Cont.)

(86) International application number:
**PCT/EP2021/059520**

(87) International publication number:
**WO 2021/209431 (21.10.2021 Gazette 2021/42)**

(54) **ABLATION PLANNING SYSTEM**

ABLATIONSPLANUNGSSYSTEM

SYSTÈME DE PLANIFICATION D'ABLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.04.2020 EP 20169279**

(43) Date of publication of application:
**22.02.2023 Bulletin 2023/08**

(73) Proprietor: **Koninklijke Philips N.V.
5656 AG Eindhoven (NL)**

(72) Inventors:
• **HAUTVAST, Guillaume Leopold Theodorus
Frederik**
**5656 AE Eindhoven (NL)**
• **SENDEN, Dave**
**5656 AE Eindhoven (NL)**
• **WAN, Win King**
**5656 AE Eindhoven (NL)**

(74) Representative: **Philips Intellectual Property &
Standards
High Tech Campus 52
5656 AG Eindhoven (NL)**

(56) References cited:
**EP-A1- 2 666 431          EP-B1- 2 666 431
US-A1- 2015 320 509**

**(Cont. next page)**

(52) Cooperative Patent Classification (CPC): (Cont.)
A61B 2090/376; A61B 2090/378

**Description**

FIELD OF THE INVENTION

The invention relates to tissue ablation system, in particular to the planning of ablations.

BACKGROUND OF THE INVENTION

In tissue ablation, an ablation probe is inserted into a subject to locally ablate tissue. Various types probes exist for ablating tissue. For example, heat, cold, radio frequency power, and lasers can all be used to ablate tissue.

United States patent application publication US20150320509A1 discloses a system for surgical procedure assistance. In one example, a first image of a patient captured prior to a surgical procedure is received. A treatment plan is generated based on the first image. The treatment plan includes information related to one or more surgical instruments. A second image of the patient captured after the surgical procedure has been initiated is received. The treatment plan is dynamically adjusted based on a pose of any of the one or more surgical instruments identified from the second image. A third image of the patient captured after a lesion is treated by at least one of the surgical instruments based on the adjusted treatment plan is received. Whether a further treatment to the lesion is needed is determined based on the third image. Upon determining a further treatment is needed, an updated treatment plan is dynamically generated based on the third image.

SUMMARY OF THE INVENTION

The invention provides for a medical instrument, a computer program, and a method in the independent claims. Embodiments are given in the dependent claims.

A difficult part of accurately performing tissue ablation is accurately planning the ablations. Embodiments may provide for a system which aids in the planning of ablations. An anatomical segmentation and a target zone segmentation are received and then used to render several three-dimensional models on a planning graphical user interface. A first three-dimensional model shows the anatomical segmentation and the target zone segmentation. A second three-dimensional model shows a remaining portion of the target zone segmentation. An ablation zone selector on the planning graphical user interface enables the selection of an ablation zone. The system then updates the remaining portion (the second three-dimensional model) by removing the ablation zone from the remaining portion. This process may be repeated to plan the entire ablation.

In one aspect the invention provides for a medical instrument that comprises a user interface. The user interface comprises a display. The medical instrument further comprises a memory storing machine-executable instructions. The medical instrument further comprises a computational system that is configured for controlling the medical instrument. The medical instrument may take different forms in different examples. In some examples the medical instrument is a workstation or computing system. In other examples the medical instrument may include other components such as an ablation system.

Execution of the machine-executable instructions causes the computational system to receive an anatomical segmentation identifying a location of an anatomical structure. The anatomical segmentation could be in the form of a segmentation of a medical image. In other examples the anatomical segmentation is simply identification of a region of the anatomical structure independent of a medical image. Execution of the machine-executable instructions further causes the computational system to receive a target zone segmentation identifying a location of a volume at least partially within the anatomical segmentation. This volume may also be referred to as the target zone.

Execution of the machine-executable instructions further causes the processor to display a planning graphical user interface using the display. The planning graphical user interface comprises a first panel configured for rendering a cross-sectional view of the anatomical segmentation and the target zone segmentation. The planning graphical user interface further comprises a second panel configured for displaying a rendering of a first three-dimensional model of the anatomical segmentation and the target zone segmentation.

The rendering of the first three-dimensional model may for example be a two-dimensional rendering of the three-dimensional model. In other examples a three-dimensional rendering may be used. The planning graphical user interface further comprises a third panel configured for rendering a second three-dimensional model of a remaining portion of the target zone segmentation. The planning graphical user interface further comprises an ablation selector configured for providing an ablation zone descriptive of a volume at least partially within the remaining portion.

Execution of the machine-executable instructions further causes the computational system to repeatedly receive the ablation zone from the ablation selector. Execution of the machine-executable instructions further causes the computational system to repeatedly update the remaining portion by removing the ablation zone from the remaining portion. This embodiment may be beneficial because it may aid in the planning of an ablation. The display of the remaining portion may aid in the selection of the proper ablation zone.

In another embodiment the ablation selector is configured to receive a selection of a volume within the remaining portion. Execution of the machine-executable instructions further causes the processor to generate the ablation zone in response to receiving the selection of a volume from the ablation selector. For example, the user interface may display the possible volumes which could be ablated.

In another embodiment the ablation selector is configured for receiving a selection of a trajectory that intersects the remaining portion. Execution of the machine-executable instructions further causes the processor to generate the ablation zone in response to receiving the selection of the trajectory from the ablation selector. A particular ablation system may have a guide or set insertion points where a probe may be inserted. The user interface may for example display the possible trajectories which a physician or other operator can choose from and can then plan the ablation and see the projected results.

In another embodiment the memory further comprises an automated planning module configured for outputting the ablation zone in response to inputting the remaining portion. The ablation selector is configured to receive an automated planning request. Execution of the machine-executable instructions further causes the processor to generate the ablation zone by inputting the remaining portion into the automated planning module in response to receiving the automated planning request. The automated planning module may for example be implemented in different ways. In one example a neural network could be used to choose the ablation zone in response to the existing remaining portion. In other examples the automated planning module may use a search algorithm that looks at all of the possible choices and then chooses an ablation zone which matches predetermined criteria. For example, the ablation zone may be chosen such that the maximum amount of tissue is ablated.

In another embodiment execution of the machine-executable instructions further causes the processor to generate insertion instructions for inserting the ablation probe in response to receiving the ablation zone from the ablation selector. The medical instrument may for example be useful in planning an ablation operation. During or after the planning has taken place the insertion instructions may be provided for use by the physician or medical technician.

In another embodiment the medical instrument comprises an ablation probe system comprising an ablation probe. The medical instrument further comprises an ablation probe tracking system registered to the anatomical segmentation. The ablation probe tracking system may for example be implemented in a variety of different ways. There may for example be a radio-frequency tag or other transmitter on the ablation probe which enables tracking. In other cases, the ablation probe tracking system may track the probe at least partially using an input medical image such as from a CT system or a magnetic resonance imaging system.

Execution of the machine-executable instructions further causes the computational system to receive probe tracking data from the ablation probe. Execution of the machine-executable instructions further causes the computational system to update the remaining portion using the probe tracking data. When the ablation probe is actually inserted into a subject the actual area which the ablation probe reaches may be different than what is intended. In this embodiment the remaining portion is updated so that it matches the actual position of the ablation probe.

In another embodiment the ablation probe is a radio-frequency ablation probe.

In another embodiment the ablation probe is a microwave ablation probe.

In another embodiment the ablation probe is a high-intensity focused ultrasound ablation probe.

In another embodiment the ablation probe is a focal or focused laser ablation probe.

In another embodiment the ablation probe is an irreversible electroporation probe.

In another embodiment the ablation probe is a cryo-ablation probe.

In another embodiment the medical instrument further comprises a guidance medical imaging system. Execution of the machine-executable instructions further causes the computational system to control the guidance medical imaging system to acquire real-time guidance medical imaging data during acquisition of tracking data from the ablation probe. Execution of the machine-executable instructions further causes the computational system to display the real-time guidance medical imaging data on the user interface in real time. The real-time guidance medical image data may for example be used to track and locate the position of the probe exactly and also update the remaining portion.

In another embodiment the guidance medical imaging system is a computed tomography system.

In another embodiment the guidance medical imaging system is an ultrasound imaging system.

In another embodiment the guidance medical imaging system is a magnetic resonance imaging system.

In another embodiment the guidance medical imaging system is an X-ray fluoroscope.

In another embodiment execution of the machine-executable instructions further causes the computational system to receive a planning magnetic resonance image descriptive of a region of interest of a subject. Anatomical segmentation identifies a location of an anatomical structure within the planning magnetic resonance image. The first panel is further configured for rendering a cross-sectional view of the planning magnetic resonance image.

In another embodiment the memory further stores an automated segmentation algorithm configured for generating the anatomical segmentation and/or the target zone segmentation in response to inputting the planning magnetic resonance image. Execution of the machine-executable instructions further causes the processor to generate the anatomical segmentation and/or the target zone segmentation by inputting the planning magnetic resonance image into the automated segmentation algorithm.

The automated segmentation algorithm may be implemented in a variety of ways. In one example the automated segmentation algorithm is implemented as a neural network. In other examples the automated segmentation algorithm may perform the segmentation by using an anatomical atlas. In another example the automated segmentation algorithm uses a deformable shape model to perform the segmentation.

In another embodiment the medical instrument further comprises a planning magnetic resonance image system configured for acquiring planning k-space data of the subject. The label planning on the planning magnetic resonance image is intended to indicate a particular magnetic resonance imaging system. Likewise, the term planning k-space data is intended to indicate specific k-space data and the word planning is used as a label. The memory further comprises planning pulse sequence commands configured for controlling the magnetic resonance imaging system to acquire the planning k-space data.

Execution of the machine-executable instructions further causes the computational system to control the planning magnetic resonance imaging system with the planning pulse sequence commands to acquire the planning k-space data. Execution of the machine-executable instructions further causes the computational system to reconstruct the planning magnetic resonance image from the planning k-space data.

In another embodiment the display is a three-dimensional display. This for example may be a display that is provided by goggles or other virtual reality or augmented reality system. Execution of the machine-executable instructions further causes the processor to render the first three-dimensional model and the second three-dimensional model three-dimensionally using the three-dimensional display.

In another aspect the invention provides for a computer program that comprises machine-executable instructions for execution by a computational system controlling a medical instrument. The medical instrument comprises a user interface comprising a display. Execution of the machine-executable instructions causes the computational system to receive an anatomical segmentation identifying a location of an anatomical structure. Execution of the machine-executable instructions further causes the computational system to receive a target zone segmentation identifying a location of a volume at least partially within the anatomical segmentation. Execution of the machine-executable instructions further causes the computational system to display a planning graphical user interface using the display.

The planning graphical user interface comprises a first panel configured for rendering a cross-sectional view of the anatomical segmentation and the target zone segmentation. The planning graphical user interface further comprises a second panel configured for displaying a rendering of the first three-dimensional model of the anatomical segmentation and the target zone segmentation. The planning graphical user interface further comprises a third panel configured for rendering a second three-dimensional model of the remaining portion of the target zone segmentation. The planning graphical user interface further comprises an ablation selector configured for providing an ablation zone descriptive of a volume at least partially within the remaining portion.

Execution of the machine-executable instructions further causes the computational system to repeatedly receive the ablation zone from the ablation selector. Execution of the machine-executable instructions further causes the computational system to repeatedly update the remaining portion by removing the ablation zone from the remaining portion.

In another aspect the invention provides for a method of operating a medical instrument according to claim 15.

The medical instrument comprises a user interface. The user interface comprises a display. The method comprises receiving an anatomical segmentation identifying a location of an anatomical structure. The method further comprises receiving a target zone segmentation identifying a location of a volume at least partially within the anatomical segmentation. The method further comprises displaying a planning graphical user interface using the display. The planning graphical user interface comprises a first panel configured for rendering a cross-sectional view of the anatomical segmentation and the target zone segmentation.

The planning graphical user interface further comprises a second panel configured for displaying a rendering of a first three-dimensional model of the anatomical segmentation and the target zone segmentation. The planning graphical user interface further comprises a third panel configured for rendering a second three-dimensional model of a remaining portion of the target zone segmentation. The planning graphical user interface further comprises an ablation selector configured for providing an ablation zone descriptive of a volume at least partially within the remaining portion. The method further comprises repeatedly receiving the ablation zone from the ablation selector. The method further comprises repeatedly updating the remaining portion by removing the ablation zone from the remaining portion.

It is understood that one or more of the aforementioned embodiments of the invention may be combined as long as the combined embodiments are not mutually exclusive.

As will be appreciated by one skilled in the art, aspects of the present invention may be embodied as an apparatus, method according to claim 15 or computer program product.

Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly comprising more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device." A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image, MR image, or magnetic resonance imaging data is defined herein as being the reconstructed two- or three-dimensional visualization of anatomic data comprised within the magnetic resonance imaging data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical instrument;
Fig. 2 illustrates a further example of a medical instrument;
Fig. 3 shows a flow chart which illustrates a method of operating the medical instrument of Fig. 1 or Fig. 2;
Fig. 4 illustrates a further example of a medical instrument;
Fig. 5 illustrates a further example of a medical instrument;
Fig. 6 illustrates an example of a planning magnetic resonance image;
Fig. 7 shows a ultrasound image with the segmentations of Fig. 6;
Fig. 8 shows a rendering of the second three-dimensional model;

Fig. 9 shows a further rendering of the second three-dimensional model; and

Fig. 10 shows a rendering of a transperineal grid template.

DETAILED DESCRIPTION OF THE EMBODIMENTS

Like numbered elements in these figures are either equivalent elements or perform the same function. Elements which have been discussed previously will not necessarily be discussed in later figures if the function is equivalent.

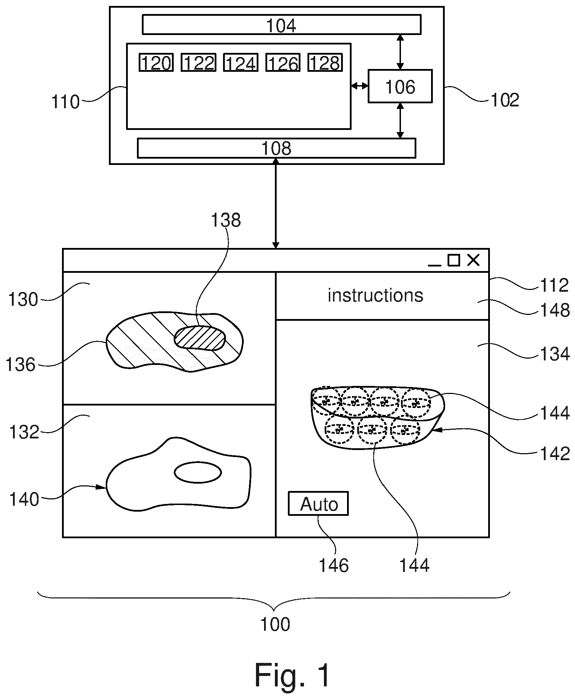

Fig. 1 illustrates an example of a medical instrument 100. The medical instrument 100 is shown as comprising in this particular example a computer 102. The computer 102 is shown as comprising an optional hardware interface 104. The hardware interface 104 may for example be used to control other components of the medical instrument 100 if they are present. The medical instrument 100 is further shown as comprising a computational system 106. The computational system 106 is intended to represent one or more computational systems or devices such as processors of other computers. The computational system 106 may be distributed also in multiple locations. The medical instrument 100 is further shown as comprising a user interface 108. The user interface comprises a planning graphical user interface 112. The medical instrument 100 is further shown as comprising a memory 110. The memory 110 represents any memory that is accessible to the computational system 106.

The medical instrument 100 illustrated in Fig. 1 may be a component or part of a variety of different types of systems. In one example the medical instrument 100 is a workstation or a computing device which is used for planning. In another example the medical instrument 100 may be integrated with an ablation probe or ablation probe system. In another example the medical instrument 100 may be integrated with a magnetic resonance imaging or other medical imaging system.

The memory 110 is further shown as comprising an optional automated planning module 126. The automated planning module may be configured for outputting a selected ablation zone in response to inputting the remaining portion. The memory 128 is further shown as comprising insertion instructions 128 which may be presented on an optional display for insertion instructions 148. For example, the insertion instructions 128 may comprise instructions on where and how far to insert an ablation probe.

The planning graphical user interface 112 is shown as comprising a first panel 130, a second panel 132, and a third panel 134. The first panel is configured for rendering a cross-sectional view of the anatomical segmentation 136 and a cross-sectional view of the target zone segmentation 138. The first panel 130 may also be configured for displaying a cross-sectional of a medical image such as a magnetic resonance image with these two cross-sectional views of the segmentations 136, 138.

The second panel 132 is configured for displaying a rendering of a first three-dimensional model 140. The first three-dimensional model 140 is a three-dimensional model of the anatomical segmentation 122 and the target zone segmentation 124. The second panel 132 may be useful because it may display the three-dimensional models of the segmentations 122 and 124 without any other medical imaging data and also in the three-dimensional means.

The third panel 134 displays a second three-dimensional model 142 that shows a remaining portion of the target zone segmentation 124. Shown within the panel 134 are a number of ablation zone selectors 144 that are volumes. These correspond to volumes that the operator can select to further ablate the target zone 124. After an ablation zone selector 144 has been removed a variety of actions may take place. For example, this area may be removed from the target zone segmentation 124 to show a smaller volume or region that still needs to be ablated. It may also cause the insertion instructions 128 to be generated. In some examples this medical instrument 100 may be used purely for planning purposes. For example, the insertion instructions 128 could be followed at a later time. In other examples the medical instrument 100 could be integrated with an ablation probe and/or medical imaging system for tracking and real-time updating of the remaining portion 142.

Also shown on the planning graphical user interface 112 is an optional automated planning request control 146. For example, when this button 146 is activated by the operator the automated planning module 126 may for example choose one of the ablation zone selectors 144 automatically.

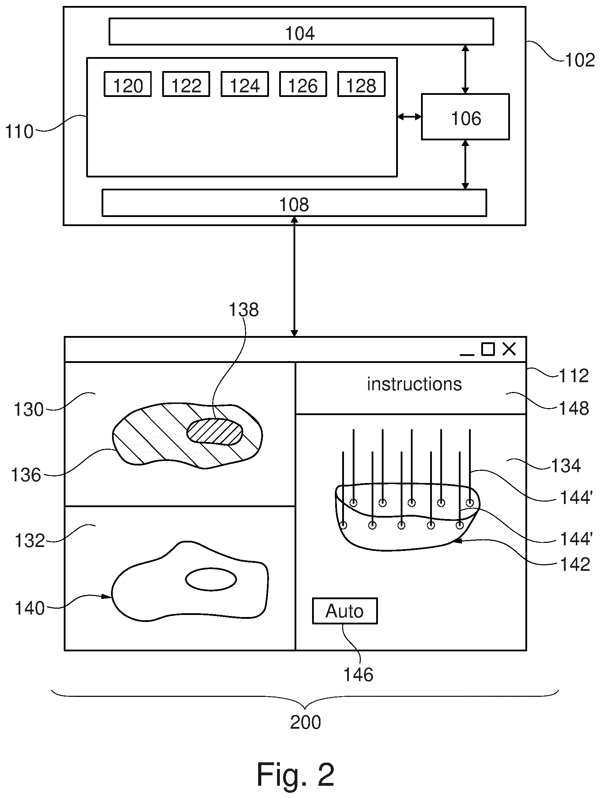

Fig. 2 illustrates a further example of a medical instrument 200. The medical instrument 200 in Fig. 2 is similar to the medical instrument 100 in Fig. 1 except in this example instead of the ablation zone selector selecting volumes the ablation zone selector instead selects trajectories 144'. These for example may be a choice of different insertion points for an ablation probe. Once a trajectory 144' is selected the ablation zone that will be ablated may be determined and this may be removed or subtracted from the remaining portion 142.

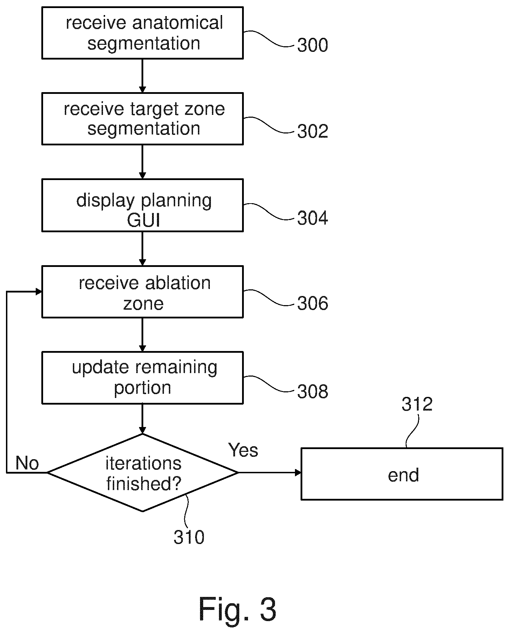

Fig. 3 shows a flowchart which illustrates a method of operating the medical instrument 100 of Fig. 1 or 200 of Fig. 2. First in step 300 the anatomical segmentation 122 identifying a location of an anatomical structure is received. Next in step 302 the target zone segmentation 124 is received and this identifies the location of a volume at least partially within the anatomical segmentation. Next in step 304 the planning graphical user interface 112 is displayed. The planning graphical user interface 112 comprises a first panel 130 that is configured for rendering a cross-sectional view of the anatomical segmentation 136 and a cross-sectional view of the target zone segmentation 138. The planning graphical user interface 112 further comprises a second panel 132 that is configured for displaying a rendering of a first three-dimensional model 140 of the anatomical segmentation 122 and the target zone segmentation 124.

The planning graphical user interface 112 further comprises a third panel 134 which is configured for rendering a second three-dimensional model 142 of a remaining portion of the target zone segmentation. The planning graphical user interface further comprises an ablation selector 144, 144' configured for providing an ablation zone descriptive of a volume at least partially within the remaining portion. The method then proceeds to step 306. In step 306 the ablation zone is received from the ablation zone selector 144, 144'. Then in step 308 the remaining portion 142 is updated by removing the ablation zone from the remaining portion. This causes the remaining portion to become smaller. The method then proceeds to decision box 310. In this step it asks are the iterations finished. If the answer is no then the method returns back to step 306 and another ablation zone is selected. If the answer is yes then the method proceeds to step 312 and the method illustrated in Fig. 3 ends.

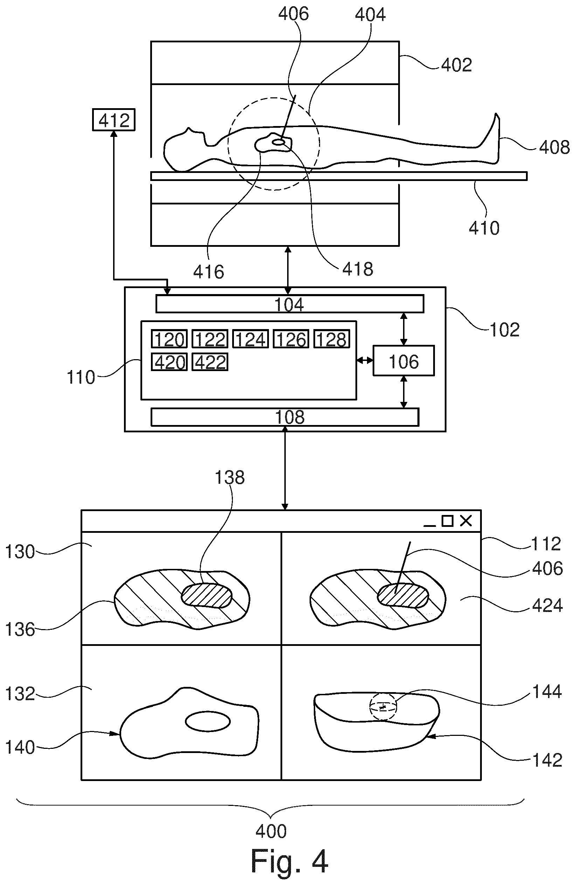

Fig. 4 shows a further example of a medical instrument 400. The medical instrument 400 in Fig. 4 is similar to the medical instruments 100 and 200 depicted in Figs. 1 and 2. The medical instrument 400 additionally comprises a guidance medical image system 402 and an ablation probe tracking system 412. There is also an ablation probe system 406 displayed. The guidance medical image system 402 may represent any number of different modalities of medical imaging that may be used for tracking the insertion of the ablation probe 406. The guidance medical imaging system 402 has an imaging zone 404 from which guidance medical image data 422 can be acquired. A subject 408 is shown as on a subject support 410 and is supported such that the anatomical structure 416 and the target zone 418 are within the imaging zone 404.

The planning graphical user interface 112 is further shown as having a real-time rendering 424 of the guidance medical image data 422 acquired by the guidance medical imaging system 402. It clearly displays the position of the ablation probe 406. The medical instrument 400 is also shown as comprising an ablation probe tracking system 412. This may for example comprise electronics which are able to localize the position and orientation of the ablation probe 406 so that is can be better determined what region of the subject 408 is actually ablated by the probe 406. This can be used to update or correct the remaining portion 142.

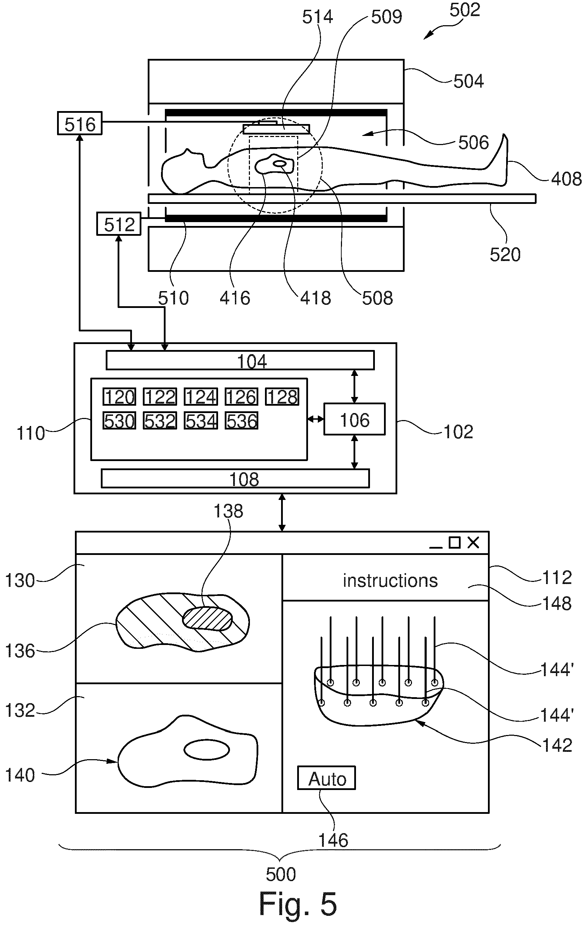

Fig. 5 illustrates a further example of a medical instrument 500. The medical instrument 500 in Fig. 5 is similar to the medical instruments 100 and 200 except that the medical instrument 500 further comprises a planning magnetic resonance imaging system 502. The planning magnetic resonance imaging system 502 is a magnetic resonance imaging system. The term planning in the planning magnetic resonance imaging system is simply a label. Likewise, the planning label is used for pulse sequence commands, k-space data and images from this planning magnetic resonance imaging system 502. The features of Fig. 5 may be freely combined with the features of Fig. 4. In some instances, the planning magnetic resonance imaging system 502 may be identical with the guidance medical imaging system 402.

The planning magnetic resonance imaging system 502 comprises a magnet 504. The magnet 504 is a superconducting cylindrical type magnet with a bore 506 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the isoplane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 506 of the cylindrical magnet 504 there is an imaging zone 508 where the magnetic field is strong and uniform enough to perform magnetic resonance imaging. A region of interest 509 is shown within the imaging zone 508. The k-space data that is acquired typically acquried for the region of interest. The subject 408 is shown as being supported by a subject support 520 such that at least a portion of the subject 408 is within the imaging zone 508 and the region of interest 509. The anatomical structure 416 and the target zone 418 are within the field of view 509 which is also inside of the imaging zone 508.

Within the bore 506 of the magnet there is also a set of magnetic field gradient coils 510 which is used for acquisition of preliminary magnetic resonance data to spatially encode magnetic spins within the imaging zone 508 of the magnet 504. The magnetic field gradient coils 510 connected to a magnetic field gradient coil power supply 512. The magnetic field gradient coils 510 are intended to be representative. Typically magnetic field gradient coils 510 comprise three separate sets of coils for spatially encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging zone 508 is a radio-frequency coil 514 for manipulating the orientations of magnetic spins within the imaging zone 508 and for receiving radio transmissions from spins also within the imaging zone 508. The radio frequency antenna may comprise multiple coil elements. The radio frequency antenna may also be referred to as a channel or antenna. The radio-frequency coil 514 is connected to a radio frequency transceiver 516. The radio-frequency coil 514 and radio frequency transceiver 516 may be replaced by separate transmit and receive coils and a separate transmitter and receiver. It is understood that the radio-frequency coil 514 and the radio frequency transceiver 516 are representative. The radio-frequency coil 514 is intended to also represent a dedicated transmit antenna and a dedicated receive antenna. Likewise the transceiver 516 may also represent a separate transmitter and receivers. The radio-frequency coil 514 may also have multiple receive/transmit elements and the radio frequency transceiver 516 may have multiple receive/transmit channels. For example if a parallel imaging technique such as SENSE is performed, the radio-frequency could 514 will have multiple coil elements.

The transceiver 516 and the gradient controller 512 are shown as being connected to the hardware interface 104 of a computer system 102.

The memory 110 is further shown as comprising planning pulse sequence commands 530. The planning pulse sequence commands 530 are pulse sequence commands. The planning pulse sequence commands 530 are data or commands which may be converted into commands that control the planning magnetic resonance imaging system 502 to acquire the planning k-space data 532. The planning k-space data 532 is k-space data. The memory 110 is further shown as comprising a planning magnetic resonance image 534. The planning magnetic resonance image was reconstructed from the planning k-space data 532 and may be segmented. The memory 110 is further shown as comprising an automated segmentation algorithm 536 that is able to automatically generate the anatomical segmentation 122 and/or the target zone segmentation 124 using the planning magnetic resonance image 534 as input. In some cases, these segmentations may also be provided manually using the planning graphical user interface 112.

In thermal tumor ablation procedures (and may other types of ablations), it is beneficial to fully cover the tumor to eradicate the disease, without ablating surrounding critical structures. For this purpose, it is possible to create a plan upfront, yet real-time feedback mechanisms while placing the ablation applicators and performing the ablation are missing.

Examples may provide a feedback mechanism to help clinicians in assessing coverage of the tumor, and especially in identifying areas that are not treated within the tumor. The system includes visualization of the untreated area in 2D and 3D including associated interaction mechanisms.

Examples may be particularly relevant to the field of thermal ablation in general, and specifically addresses the need to support identification of areas that are not treated. Although the disclosure below is also relevant to many other types of ablation.

Percutaneous thermal ablation is an interventional cancer treatment option that has seen significant increase in adoption in the past decade, and is predicted to continue to grow at a CAGR of 8-10% through 2024. Thermal ablation can be delivered using various ablation modalities, including radiofrequency (RF), microwave (MW), high-intensity focused ultrasound (HIFU), focal laser ablation (FLA), irreversible electroporation (IRE), cryo-ablation, etc.

In clinical practice, these ablation procedures consist of placing one or more ablation applicators (ablation probe 406) inside or near the target region (target zone 418) with the help of image guidance. Typically, physicians place these needle-like applicators while inspecting real-time ultrasound or interventional radiology images (CT/MR), based on information provided from the manufacturer, results in clinical trials and personal experience. The use of more advanced ablation therapy planning systems (ATPS) to plan the ablation and guide needle placement, akin to the radiation therapy planning systems (RTPS) used in brachytherapy procedures, is not wide spread due to their limited availability.

In current ablation procedures, quality assurance is limited. Most procedures are performed without a plan, and if a plan is defined, it is visualized by displaying the covered/treated area, without direct feedback on the presence of small untreated areas ("gaps") between individual ablations inside the target.

Examples may provide for an ablation therapy guidance system, capable displaying the untreated area within the target (the remaining portion 142). This display (planning graphical user interface 112) is interactive, 3D rendering that allows the user to determine where to place additional applicators to cover the untreated areas.

Given discrete binary representations of a lesion L (target zone 418) to be treated and the ablation zone Z (600), the untreated area U (the remaining portion 142) can be computed by the following equation:

$$U = L \backslash Z = \{x \in L \mid x \notin Z\} \tag{1}$$

For the purpose of visualization, the binary region U may need to be converted in a mesh or contour structure. For this purpose, the calculation may include marching squares or marching cubes as a post-processing step.

The untreated area may be visualized in 2D, e.g. on top of multi-planar reformat (MPR) visualizations of 3D image volume covering the area to be treated, or on top of live US images that are registered to the applicator plan through real-time tracking.

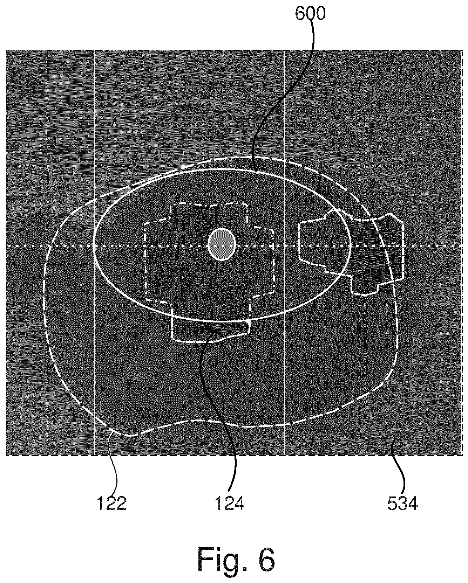

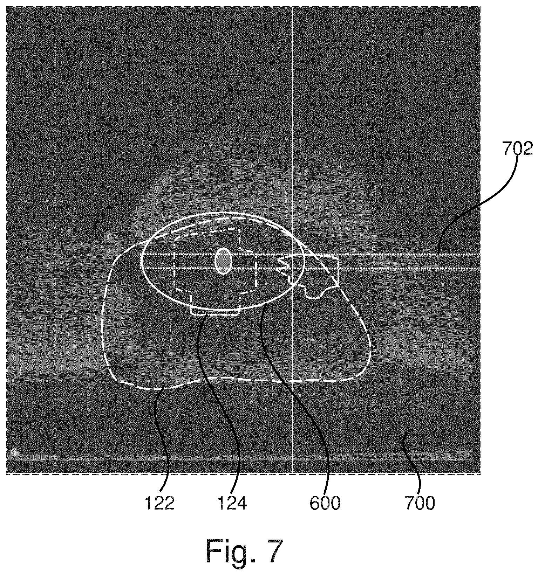

Figs. 6 and 7 below provide example of 2D untreated area visualizations. Fig. 6 shows an example of a planning magnetic resonance image 534. There is visible an anatomical segmentation 122 with a target zone segmentation 124 and an ablation zone 600.

Fig. 7 shows the same segmentations 122, 124 and the location of the ablation zone 600 in an ultrasound image 700. The location of a suggested position for the ablation probe 702 is also displayed.

The untreated area (the remaining portion 142) may be visualized in 3D, e.g.by shaded surface in combination with other anatomical parts to reveal the relative location of the untreated area with respect to surrounding tissue.

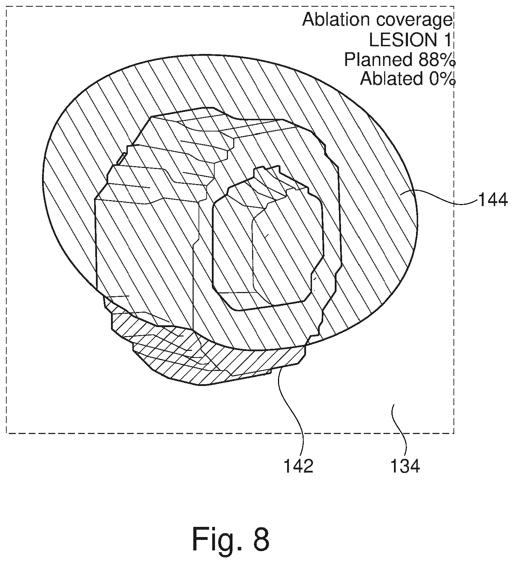

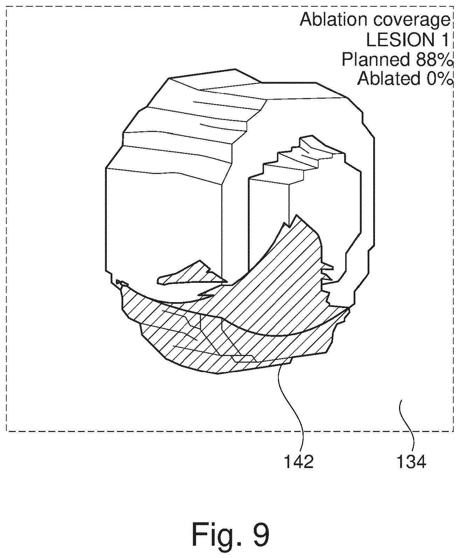

Figs. 8 and 9 show two views of the third panel 134. In Fig. 8 the remaining portion 142 is the entire target zone segmentation 124. An ablation zone 144 has been selected. After this is selected the volume of the ablation zone is removed from the remaining portion 142. Fig. 9 shows the remaining portion 142 after the ablation zone 144 has been removed.

In transperineal prostate procedures, the visualization may include a visualization of the transperineal grid template (a needle guidance device) through which applicators are being inserted. This enables the users to decide on the correct approach to cover the untreated area.

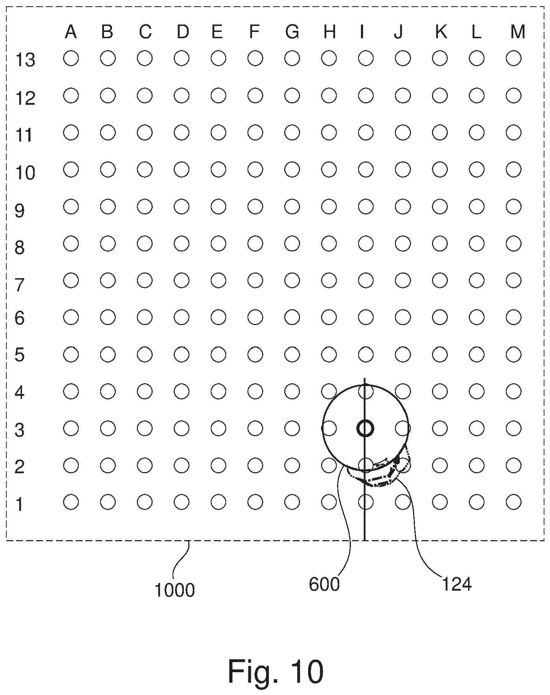

Fig. 10 shows an alternative means of visualization. Illustrated in Fig. 10 is a rendering of a transperineal grid template that lays a matrix of circles labeled A-M and 1-13. These represent different locations where an ablation probe may be inserted. Underneath this grid 1000 the ablation zone 600 is illustrated as well as the remaining portion 124. Superimposing 600 in 124 on the grid 1000 may help an operator visualize the proper location to insert an ablation probe.

When including more regions for healthy tissue and organs at risk, these 3D surface renderings may possibly be generated with the help of advanced techniques, such as glass rendering.

When the untreated area is visualized in 3D renderings, alongside MPR visualizations of a 3D image volume of the area to be treated, the system may incorporate a navigation aid that positions the MPR viewers based on a clicked untreated area as visualized in a 3D rendering.

In some examples, when the untreated area is visualized, the user may be able to plan an ablation within the untreated area:

by clicking a location in the untreated area (in MPR view or 3D rendering),
by clicking a needle trajectory that passes through the untreated area (e.g. .clicking a grid hole in prostate procedures), and
by clicking a button to initiate automated planning.

The visualization of the untreated areas plays an important role during two decision moments in an ablation procedure that is guided aby an ATPS.

The first moment is the review and approval of the plan that is to be implanted by the user. Before starting to do so, the user can inspect his plan to assess whether there are no remaining uncovered areas in the target lesion.

The second moment is after placement of the applicators, where small deviations from the plan are inevitable. Such deviations may introduce small untreated areas in between the applicators, which can now easily be visualized. If there are gaps, the user can decide to plan further ablations, to place an applicator in the untreated area, or to accept the gap as is.

Examples may possibly provide for medical instruments with one or more of the following features:
A medical instrument cable of computing and displaying the untreated area within a target for an ablation treatment.

A medical instrument capable of visualizing the untreated area relative to needle guidance device.

A medical instrument capable of calculating a new ablation plan that will cover the untreated area.

A medical instrument in which the display of the untreated area is achieved using 2D rendering techniques.

A medical instrument in which the display of the untreated area is achieved using 3D rendering techniques.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| | |
|---|---|
| 100 | medical instrument |
| 102 | computer |
| 104 | hardware interface |
| 106 | computational system |
| 108 | user interface |
| 110 | memory |
| 112 | planning graphical user interface |
| 120 | machine executable instructions |
| 122 | anatomical segmentation |
| 124 | target zone segmentation |
| 126 | automated planning module |
| 128 | insertion instructions |
| 130 | first panel |
| 132 | second panel |
| 134 | third panel |
| 136 | cross sectional view of anatomical segmentation |
| 138 | cross sectional view of target zone segmentation |
| 140 | first three-dimensional model |
| 142 | second three-dimensional model of the remaining portion |
| 144 | ablation zone selector (volume selector) |
| 144' | ablation zone selector (trajectory selector) |
| 146 | automated planning request control |
| 148 | display for insertion instructions |
| 200 | medical instrument |
| 300 | receive an anatomical segmentation identifying a location of an anatomical structure |
| 302 | receive a target zone segmentation identifying a location of a volume at least partially within the anatomical segmentation |
| 304 | display a planning graphical user interface using the display |
| 306 | receive the ablation zone from the ablation selector |
| 308 | update the remaining portion by removing the ablation zone from the remaining portion |
| 310 | Iterations finished? |
| 312 | end |
| 400 | medical instrument |
| 402 | guidance medical imaging system |
| 404 | imaging zone |
| 406 | ablation probe system |
| 408 | subject |
| 410 | support |
| 412 | ablation probe tracking system |
| 416 | anatomical structure |
| 418 | target zone |
| 420 | probe tracking data |
| 422 | guidance medical image data |
| 424 | real time rendering of guidance medical image data |
| 500 | medical instrument |
| 502 | planning magnetic resonance imaging system |
| 504 | magnet |
| 506 | bore of magnet |
| 508 | imaging zone |
| 509 | region of interest |
| 510 | magnetic field gradient coils |
| 512 | magnetic field gradient coil power supply |
| 514 | radio-frequency coil |
| 516 | transceiver |
| 520 | subject support |
| 530 | planning pulse sequence commands |
| 532 | planning k-space data |
| 534 | planning magentic resonance image |

| 536 | automated segmentation algorithm |
| 600 | ablation zone700 ultrasound image |
| 702 | location of ablation probe |
| 1000 | transperineal grid template |

**Claims**

1. A medical instrument (100, 200, 400, 500) comprising:

   - a user interface (108) comprising a display;
   - a memory (110) storing machine executable instructions (120);
   - a computational system (106) configured for controlling the medical instrument, wherein execution of the machine executable instructions causes the computational system to:
   - receive (300) an anatomical segmentation (122) identifying a location of an anatomical structure (416);
   - receive (302) a target zone segmentation (124) identifying a location of a volume (418) at least partially within the anatomical segmentation; and
   - display (304) a planning graphical user interface (112) using the display; wherein the planning graphical user interface comprises a first panel (130) configured for rendering a cross sectional view of the anatomical segmentation (136) and the target zone segmentation (138); wherein the planning graphical user interface further comprises a second panel (132) configured for displaying a rendering of a first three-dimensional model (140) of the anatomical segmentation and the target zone segmentation;

   wherein the planning graphical user interface further comprises a third panel (134) configured for rendering a second three-dimensional model (142) of a remaining portion of the target zone segmentation; wherein the planning graphical user interface further comprises an ablation selector (144, 144', 146) configured to provide a user-selectable ablation zone, descriptive of a volume at least partially within the remaining portion that the user can select to ablate the target zone segmentation;

   wherein execution of the machine executable instructions further causes the computational system to iteratively :

   - receive (306) an ablation zone from the ablation selector (144, 144', 146); and
   - update (308) the remaining portion by removing the ablation zone from the remaining portion, until the corresponding iterations are finished.

2. The medical instrument of claim 1, wherein the ablation selector is configured to receive a selection of a volume (144) within the remaining portion, wherein execution of the machine executable instructions further causes the processor to generate the ablation zone in response to receiving the selection of the volume from the ablation selector.

3. The medical instrument of claim 1 or 2, wherein the ablation selector is configured to receive a selection of a trajectory (144') that intersects the remaining portion, wherein execution of the machine executable instructions further causes the processor to generate the ablation zone in response to receiving the selection of the trajectory from the ablation selector.

4. The medical instrument of claim 1, 2, or 3, wherein the memory further comprises an automated planning module (126) configured for outputting the ablation zone in response to inputting the remaining portion, wherein the ablation selector is configured to receive an automated planning request, wherein execution of the machine executable instructions further causes the processor to generate the ablation zone by inputting the remaining portion into the automated planning module in response to receiving the automated planning request.

5. The medical instrument of any one of the preceding claims, wherein execution of the machine executable instructions further causes the processor to generate insertion instructions (128) for inserting the ablation probe in response to receiving the ablation zone from the ablation selector.

6. The medical instrument of any one of the preceding claims, wherein the medical instrument comprises an ablation probe system (406) comprising an ablation probe, wherein the medical instrument further comprises an ablation probe tracking system (420) registered to the anatomical segmentation, wherein execution of the machine executable instructions further causes the computational system to:

   - receive probe tracking data (420) from the ablation probe; and
   - update the remaining portion using the probe tracking data.

7. The medical instrument of claim 6, wherein the ablation probe is any one of the following: a radio frequency ablation probe, a microwave ablation probe, a high-intensity focused ultrasound ablation probe, a focal laser ablation probe, an irreversible electroporation probe, and a cryo-ablation probe.

8. The medical instrument of any one of claims 6 or 7, wherein the medical instrument further comprises a guidance medical imaging system (402), wherein execution of the machine executable instructions further causes the computational system to:

   - control the guidance medical imaging system to acquire real-time guidance medical image data (422) during acquisition of tracking data from the ablation probe; and
   - display the real-time guidance medical image data on the user interface in real time.

9. The medical instrument of claim 8, wherein the guidance medical imaging system is any one of the following: a computed tomography system, an ultrasound imaging system, a magnetic resonance imaging system, and an X-ray fluoroscope.

10. The medical instrument of any one of the preceding claims, wherein execution of the machine executable instructions causes the computational system to receive a planning magnetic resonance image (534) descriptive of a region of interest of a subject (408), wherein the anatomical segmentation identifies a location of the anatomical structure within the planning magnetic resonance image, wherein the first panel is further configured for rendering a cross sectional view of the planning magnetic resonance image.

11. The medical instrument of claim 10, wherein the memory further stores an automated segmentation algorithm (536) configured for generating the anatomical segmentation and/or the target zone segmentation in response to inputting the planning magnetic resonance image, wherein execution of the machine executable instructions further causes the processor to generate the anatomical segmentation and/or the target zone segmentation by inputting the planning magnetic resonance image into the automated segmentation algorithm.

12. The medical instrument of claim 10 or 11, wherein the medical instrument further comprises a planning magnetic resonance imaging system (502) configured for acquiring planning k-space data (532) descriptive of the subject, wherein the memory further comprises planning pulse sequence commands (530) configured for controlling the magnetic resonance imaging system to acquire the planning k-space data, wherein execution of the machine executable instructions further causes the computational system to:

   - control the planning magnetic resonance imaging system with the planning pulse sequence commands to acquire the planning k-space data; and
   - reconstruct the planning magnetic resonance image from the planning k-space data.

13. The medical instrument of any one of the preceding claims, wherein the display is a three-dimensional display, wherein execution of the machine executable instructions further causes the processor to render the first three-dimensional model and the second three-dimensional model three-dimensionally using the three-dimensional display.

14. A computer program comprising machine executable instructions (120) for execution by a computational system (106) controlling a medical instrument (100, 200, 400, 500), wherein the medical instrument comprises a user interface (108) comprising a display; wherein execution of the machine executable instructions causes the computational system to:

   - receive (300) an anatomical segmentation (122) identifying a location of an anatomical structure (416);
   - receive (302) a target zone segmentation identifying (124) a location of a volume (418) at least partially within the anatomical segmentation; and
   - display (304) a planning graphical user interface (112) using the display;
   wherein the planning graphical user interface comprises a first panel configured for rendering a cross sectional view of the anatomical segmentation (136) and a cross sectional view of the target zone segmentation (138); wherein the planning graphical user interface further comprises a second panel (132) configured for displaying a rendering of a first three-dimensional model (140) of the anatomical segmentation and the target zone segmentation; wherein the planning graphical user interface further comprises a third panel (134) configured for rendering a second three-dimensional model (142) of a remaining portion of the target zone segmentation;

wherein the planning graphical user interface further comprises an ablation selector (144, 144', 146) configured to provide a user-selectable ablation zone descriptive of a volume at least partially within the remaining portion that the user can select to ablate the target zone segmentation;

wherein execution of the machine executable instructions further causes the computational system to iteratively :

- receive (306) an ablation zone from the ablation selector (144, 144', 146); and
- update (308) the remaining portion by removing the ablation zone from the remaining portion, until the corresponding iterations are finished.

15. A non-therapeutic and non-surgical

method of operating a medical instrument (100, 200, 400, 500), wherein the medical instrument comprises a user interface (108), wherein the user interface comprises a display,

wherein the method comprises:

- receiving (300) an anatomical segmentation (122) identifying a location of an anatomical structure (416);
- receiving (302) a target zone segmentation (124) identifying a location of a volume (416) at least partially within the anatomical segmentation; and
- displaying (304) a planning graphical user interface (112) using the display;

wherein the planning graphical user interface comprises a first panel (130) configured for rendering a cross sectional view of the anatomical segmentation (136) and the target zone segmentation (138); wherein the planning graphical user interface further comprises a second panel (132) configured for displaying a rendering of a first three-dimensional model (140) of the anatomical segmentation and the target zone segmentation; wherein the planning graphical user interface further comprises a third panel (134) configured for rendering a second three-dimensional model (142) of a remaining portion of the target zone segmentation; wherein the planning graphical user interface further comprises an ablation selector (144, 144', 146) configured to provide a user-selectable ablation zone,

descriptive of a volume at least partially within the remaining portion that the user can select to ablate the target zone segmentation;

wherein the method comprises iteratively :

- receiving (306) an ablation zone from the ablation selector (144, 144', 146); and
- updating (308) the remaining portion by removing the ablation zone from the remaining portion, until the corresponding iterations are finished.

**Patentansprüche**

1. Medizinisches Instrument (100, 200, 400, 500), umfassend:

- eine Benutzeroberfläche (108), die eine Anzeige umfasst;
- einen Speicher (110), der maschinenausführbare Anweisungen (120) speichert;
- ein Rechensystem (106), das dafür konfiguriert ist, das medizinische Instrument zu steuern, wobei die Ausführung der maschinenausführbaren Anweisungen bewirkt, dass das Rechensystem:
- eine anatomische Segmentierung (122) empfängt (300), die eine Position einer anatomischen Struktur (416) identifiziert;
- eine Zielzonensegmentierung (124) empfängt (302), die eine Position eines Volumens (418) identifiziert, das mindestens teilweise innerhalb der anatomischen Segmentierung liegt; und
- unter Verwendung der Anzeige eine grafische Planungsbenutzeroberfläche (112) anzeigt (304); wobei die grafische Planungsbenutzeroberfläche ein erstes Bedienfeld (130) umfasst, das dafür konfiguriert ist, eine Querschnittsansicht der anatomischen Segmentierung (136) und der Zielzonensegmentierung (138) zu rendern; wobei die grafische Planungsbenutzeroberfläche weiter ein zweites Bedienfeld (132) umfasst, das dafür konfiguriert ist, ein Rendering eines ersten dreidimensionalen Modells (140) der anatomischen Segmentierung und der Zielzonensegmentierung anzuzeigen; wobei die grafische Planungsbenutzeroberfläche weiter ein drittes Bedienfeld (134) umfasst, das dafür konfiguriert ist, ein zweites dreidimensionales Modell (142) eines verbleibenden Abschnitts der Zielzonensegmentierung zu rendern; wobei die grafische Planungsbenutzeroberfläche weiter einen Ablationsselektor (144, 144', 146) umfasst, der dazu konfiguriert ist, eine vom Benutzer auswählbare Ablationszone bereitzustellen, die ein Volumen beschreibt, das mindestens teilweise innerhalb des verbleibenden Abschnitts liegt, die der Benutzer auswählen kann, um die Zielzonensegmentierung zu ablatieren;

wobei die Ausführung der maschinenausführbaren Anweisungen weiter bewirkt, dass das Rechensystem iterativ:

- vom Ablationsselektor (144, 144', 146) eine Ablationszone empfängt (306); und
- den verbleibenden Abschnitt durch Entfernen der Ablationszone vom verbleibenden Abschnitt aktualisiert (308), bis die entsprechenden Iterationen abgeschlossen sind.

2. Medizinisches Instrument nach Anspruch 1, wobei der Ablationsselektor dazu konfiguriert ist, eine Auswahl eines Volumens (144) innerhalb des verbleibenden Abschnitts zu empfangen, wobei die Ausführung der maschinenausführbaren Anweisungen weiter bewirkt, dass der Prozessor die Ablationszone in Reaktion auf das Empfangen der Auswahl des Volumens vom Ablationsselektor erzeugt.

3. Medizinisches Instrument nach Anspruch 1 oder 2, wobei der Ablationsselektor dazu konfiguriert ist, eine Auswahl einer Trajektorie (144') zu empfangen, die den verbleibenden Abschnitt schneidet, wobei die Ausführung der maschinenausführbaren Anweisungen weiter bewirkt, dass der Prozessor die Ablationszone in Reaktion auf das Empfangen der Auswahl der Trajektorie vom Ablationsselektor erzeugt.

4. Medizinisches Instrument nach Anspruch 1, 2 oder 3, wobei der Speicher weiter ein automatisiertes Planungsmodul (126) umfasst, das dafür konfiguriert ist, die Ablationszone in Reaktion auf das Eingeben des verbleibenden Abschnitts auszugeben, wobei der Ablationsselektor dazu konfiguriert ist, eine automatisierte Planungsanforderung zu empfangen, wobei die Ausführung der maschinenausführbaren Anweisungen weiter bewirkt, dass der Prozessor die Ablationszone durch Eingeben des verbleibenden Abschnitts in das automatisierte Planungsmodul in Reaktion auf das Empfangen der automatisierten Planungsanforderung erzeugt.

5. Medizinisches Instrument nach einem der vorstehenden Ansprüche, wobei die Ausführung der maschinenausführbaren Anweisungen weiter bewirkt, dass der Prozessor Einführanweisungen (128) zum Einführen der Ablationssonde in Reaktion auf das Empfangen der Ablationszone vom Ablationsselektor erzeugt.

6. Medizinisches Instrument nach einem der vorstehenden Ansprüche, wobei das medizinische Instrument ein Ablationssondensystem (406) umfasst, das eine Ablationssonde umfasst, wobei das medizinische Instrument weiter ein Ablationssonden-Verfolgungssystem (420) umfasst, das für die anatomische Segmentierung registriert ist, wobei die Ausführung der maschinenausführbaren Anweisungen weiter bewirkt, dass das Rechensystem:

- Sondenverfolgungsdaten (420) von der Ablationssonde empfängt; und
- den verbleibenden Abschnitt unter Verwendung der Sondenverfolgungsdaten aktualisiert.

7. Medizinisches Instrument nach Anspruch 6, wobei die Ablationssonde eine der Folgenden ist: eine Radiofrequenz-Ablationssonde, eine Mikrowellen-Ablationssonde, eine hochintensive fokussierte Ultraschall-Ablationssonde, eine fokale Laser-Ablationssonde, eine irreversible Elektroporationssonde und eine Kryo-Ablationssonde.

8. Medizinisches Instrument nach einem der Ansprüche 6 oder 7, wobei das medizinische Instrument weiter ein medizinisches Führungsbildgebungssystem (402) umfasst, wobei die Ausführung der maschinenausführbaren Anweisungen weiter bewirkt, dass das Rechensystem:

- das medizinische Führungsbildgebungssystem steuert, um während der Erfassung von Verfolgungsdaten von der Ablationssonde medizinische Echtzeit-Führungsbilddaten (422) zu erfassen; und
- die medizinischen Echtzeit-Führungsbilddaten in Echtzeit auf der Benutzeroberfläche anzeigt.

9. Medizinisches Instrument nach Anspruch 8, wobei das medizinische Führungsbildgebungssystem eines der Folgenden ist: ein Computertomographiesystem, ein Ultraschallbildgebungssystem, ein Magnetresonanzbildgebungssystem und ein Röntgenfluoroskop.

10. Medizinisches Instrument nach einem der vorstehenden Ansprüche, wobei die Ausführung der maschinenausführbaren Anweisungen bewirkt, dass das Rechensystem ein Planungsmagnetresonanzbild (534) empfängt, das einen Bereich von Interesse eines Subjekts (408) beschreibt, wobei die anatomische Segmentierung eine Position der anatomischen Struktur innerhalb des Planungsmagnetresonanzbildes identifiziert, wobei das erste Bedienfeld weiter dafür konfiguriert ist, eine Querschnittsansicht des Planungsmagnetresonanzbildes zu rendern.

11. Medizinisches Instrument nach Anspruch 10, wobei der Speicher weiter einen automatisierten Segmentierungsalgorithmus (536) speichert, der dafür konfiguriert ist, die anatomische Segmentierung und/oder die Zielzonensegmentierung in Reaktion auf das Eingeben des Planungsmagnetresonanzbildes zu erzeugen, wobei die Ausführung der maschinenausführbaren Anweisungen weiter bewirkt, dass der Prozessor die anatomische Segmentierung und/oder die Zielzonensegmentierung durch Eingeben des Planungsmagnetresonanzbildes in den automatisierten Segmentierungsalgorithmus erzeugt.

12. Medizinisches Instrument nach Anspruch 10 oder 11, wobei das medizinische Instrument weiter ein Planungsmagnetresonanzbildgebungssystem (502) umfasst, das dafür konfiguriert ist, Planungs-K-Raum-Daten (532), die das Subjekt beschreiben, zu erfassen, wobei der Speicher weiter Planungsimpulsfolgebefehle (530) umfasst, die dafür konfiguriert sind, das Magnetresonanzbildgebungssystem zu steuern, um die Planungs-K-Raum-Daten zu erfassen, wobei die Ausführung der maschinenausführbaren Anweisungen weiter bewirkt, dass das Rechensystem:

- das Planungsmagnetresonanzbildgebungssystem mit den Planungsimpulsfolgebefehlen steuert, um die Planungs-K-Raum-Daten zu erfassen; und
- aus den Planungs-K-Raum-Daten das Planungsmagnetresonanzbild rekonstruiert.

13. Medizinisches Instrument nach einem der vorstehenden Ansprüche, wobei die Anzeige eine dreidimensionale Anzeige ist, wobei die Ausführung der maschinenausführbaren Anweisungen weiter bewirkt, dass der Prozessor das erste dreidimensionale Modell und das zweite dreidimensionale Modell unter Verwendung der dreidimensionalen Anzeige dreidimensional rendert.

14. Computerprogramm, das maschinenausführbare Anweisungen (120) zur Ausführung durch ein Rechensystem (106), das ein medizinisches Instrument (100, 200, 400, 500) steuert, umfasst, wobei das medizinische Instrument eine Benutzeroberfläche (108) umfasst, die eine Anzeige umfasst; wobei die Ausführung der maschinenausführbaren Anweisungen bewirkt, dass das Rechensystem:

- eine anatomische Segmentierung (122) empfängt (300), die eine Position einer anatomischen Struktur (416) identifiziert;
- eine Zielzonensegmentierung (124) empfängt (302), die eine Position eines Volumens (418) identifiziert, das mindestens teilweise innerhalb der anatomischen Segmentierung liegt; und
- unter Verwendung der Anzeige eine grafische Planungsbenutzeroberfläche (112) anzeigt (304); wobei die grafische Planungsbenutzeroberfläche ein erstes Bedienfeld umfasst, das dafür konfiguriert ist, eine Querschnittsansicht der anatomischen Segmentierung (136) und eine Querschnittsansicht der Zielzonensegmentierung (138) zu rendern; wobei die grafische Planungsbenutzeroberfläche weiter ein zweites Bedienfeld (132) umfasst, das dafür konfiguriert ist, ein Rendering eines ersten dreidimensionalen Modells (140) der anatomischen Segmentierung und der Zielzonensegmentierung anzuzeigen; wobei die grafische Planungsbenutzeroberfläche weiter ein drittes Bedienfeld (134) umfasst, das dafür konfiguriert ist, ein zweites dreidimensionales Modell (142) eines verbleibenden Abschnitts der Zielzonensegmentierung zu rendern; wobei die grafische Planungsbenutzeroberfläche weiter einen Ablationsselektor (144, 144', 146) umfasst, der dazu konfiguriert ist, eine vom Benutzer auswählbare Ablationszone bereitzustellen, die ein Volumen beschreibt, das mindestens teilweise innerhalb des verbleibenden Abschnitts liegt, die der Benutzer auswählen kann, um die Zielzonensegmentierung zu ablatieren;

wobei die Ausführung der maschinenausführbaren Anweisungen weiter bewirkt, dass das Rechensystem iterativ:

- vom Ablationsselektor (144, 144', 146) eine Ablationszone empfängt (306); und
- den verbleibenden Abschnitt durch Entfernen der Ablationszone vom verbleibenden Abschnitt aktualisiert (308), bis die entsprechenden Iterationen abgeschlossen sind.

15. Nicht-therapeutisches und nicht-chirurgisches Verfahren zum Betreiben eines medizinischen Instruments (100, 200, 400, 500), wobei das medizinische Instrument eine Benutzeroberfläche (108) umfasst, wobei die Benutzeroberfläche eine Anzeige umfasst,
wobei das Verfahren umfasst:

- Empfangen (300) einer anatomischen Segmentierung (122), die eine Position einer anatomischen Struktur (416) identifiziert;

- Empfangen (302) einer Zielzonensegmentierung (124), die eine Position eines Volumens (416) identifiziert, das mindestens teilweise innerhalb der anatomischen Segmentierung liegt; und

- Anzeigen (304) einer grafischen Planungsbenutzeroberfläche (112) unter Verwendung der Anzeige; wobei die grafische Planungsbenutzeroberfläche ein erstes Bedienfeld (130) umfasst, das dafür konfiguriert ist, eine Querschnittsansicht der anatomischen Segmentierung (136) und der Zielzonensegmentierung (138) zu rendern; wobei die grafische Planungsbenutzeroberfläche weiter ein zweites Bedienfeld (132) umfasst, das dafür konfiguriert ist, ein Rendering eines ersten dreidimensionalen Modells (140) der anatomischen Segmentierung und der Zielzonensegmentierung anzuzeigen; wobei die grafische Planungsbenutzeroberfläche weiter ein drittes Bedienfeld (134) umfasst, das dafür konfiguriert ist, ein zweites dreidimensionales Modell (142) eines verbleibenden Abschnitts der Zielzonensegmentierung zu rendern; wobei die grafische Planungsbenutzeroberfläche weiter einen Ablationsselektor (144, 144', 146) umfasst, der dazu konfiguriert ist, eine vom Benutzer auswählbare Ablationszone bereitzustellen, die ein Volumen beschreibt, das mindestens teilweise innerhalb des verbleibenden Abschnitts liegt, die der Benutzer auswählen kann, um die Zielzonensegmentierung zu ablatieren; wobei das Verfahren iterativ umfasst:

- Empfangen (306) einer Ablationszone vom Ablationsselektor (144, 144', 146); und
- Aktualisieren (308) des verbleibenden Abschnitts durch Entfernen der Ablationszone vom verbleibenden Abschnitt, bis die entsprechenden Iterationen abgeschlossen sind.

## Revendications

1. Instrument médical (100, 200, 400, 500) comprenant :

- une interface utilisateur (108) comprenant un affichage ;
- une mémoire (110) stockant des instructions exécutables par machine (120) ;
- un système informatique (106) configuré pour commander l'instrument médical, dans lequel l'exécution des instructions exécutables par machine amène le système informatique à :
- recevoir (300) une segmentation anatomique (122) identifiant un emplacement d'une structure anatomique (416) ;
- recevoir (302) une segmentation de zone cible (124) identifiant un emplacement d'un volume (418) au moins partiellement à l'intérieur de la segmentation anatomique ; et
- afficher (304) une interface utilisateur graphique de planification (112) à l'aide de l'affichage ; dans lequel l'interface utilisateur graphique de planification comprend un premier panneau (130) configuré pour restituer une vue en coupe transversale de la segmentation anatomique (136) et de la segmentation de zone cible (138) ; dans lequel l'interface utilisateur graphique de planification comprend en outre un deuxième panneau (132) configuré pour afficher un rendu d'un premier modèle tridimensionnel (140) de la segmentation anatomique et de la segmentation de zone cible ; dans lequel l'interface utilisateur graphique de planification comprend en outre un troisième panneau (134) configuré pour restituer un second modèle tridimensionnel (142) d'une partie restante de la segmentation de zone cible ; dans lequel l'interface utilisateur graphique de planification comprend en outre un sélecteur d'ablation (144, 144', 146) configuré pour fournir une zone d'ablation sélectionnable par l'utilisateur, descriptive d'un volume au moins partiellement à l'intérieur de la partie restante que l'utilisateur peut sélectionner pour ablater la segmentation de zone cible ;

dans lequel l'exécution des instructions exécutables par machine amène en outre le système informatique à, de manière itérative :

- recevoir (306) une zone d'ablation à partir du sélecteur d'ablation (144, 144', 146) ; et
- mettre à jour (308) la partie restante en supprimant la zone d'ablation de la partie restante, jusqu'à ce que les itérations correspondantes soient terminées.

2. Instrument médical selon la revendication 1, dans lequel le sélecteur d'ablation est configuré pour recevoir une sélection d'un volume (144) à l'intérieur de la partie restante, dans lequel l'exécution des instructions exécutables par machine amène en outre le processeur à générer la zone d'ablation en réponse à la réception de la sélection du volume à partir du sélecteur d'ablation.

3. Instrument médical selon la revendication 1 ou 2, dans lequel le sélecteur d'ablation est configuré pour recevoir une sélection d'une trajectoire (144') qui coupe la partie restante, dans lequel l'exécution des instructions exécutables par machine amène en outre le processeur à générer la zone d'ablation en réponse à la réception de la sélection de la trajectoire à partir du sélecteur d'ablation.

4. Instrument médical selon la revendication 1, 2 ou 3, dans lequel la mémoire comprend en outre un module de planification automatisée (126) configuré pour délivrer en sortie la zone d'ablation en réponse à l'entrée de la partie restante, dans lequel le sélecteur d'ablation est configuré pour recevoir une demande de planification automatisée, dans lequel l'exécution des instructions exécutables par machine amène en outre le processeur à générer la zone d'ablation en entrant la partie restante dans le module de planification automatisée en réponse à la réception de la demande de planification automatisée.

5. Instrument médical selon l'une quelconque des revendications précédentes, dans lequel l'exécution des instructions exécutables par machine amène en outre le processeur à générer des instructions d'insertion (128) pour insérer la sonde d'ablation en réponse à la réception de la zone d'ablation à partir du sélecteur d'ablation.

6. Instrument médical selon l'une quelconque des revendications précédentes, dans lequel l'instrument médical comprend un système de sonde d'ablation (406) comprenant une sonde d'ablation, dans lequel l'instrument médical comprend en outre un système de suivi de sonde d'ablation (420) enregistré sur la segmentation anatomique, dans lequel l'exécution des instructions exécutables par machine amène en outre le système informatique à :

- recevoir des données de suivi de sonde (420) à partir de la sonde d'ablation ; et
- mettre à jour la partie restante à l'aide des données de suivi de sonde.

7. Instrument médical selon la revendication 6, dans lequel la sonde d'ablation est l'une quelconque des suivantes : une sonde d'ablation par radiofréquence, une sonde d'ablation par micro-ondes, une sonde d'ablation par ultrasons focalisés de haute intensité, une sonde d'ablation laser focale, une sonde d'électroporation irréversible et une sonde de cryo-ablation.

8. Instrument médical selon l'une quelconque des revendications 6 ou 7, dans lequel l'instrument médical comprend en outre un système d'imagerie médicale de guidage (402), dans lequel l'exécution des instructions exécutables par machine amène en outre le système informatique à :

- commander le système d'imagerie médicale de guidage pour acquérir des données d'image médicale de guidage en temps réel (422) pendant l'acquisition de données de suivi à partir de la sonde d'ablation ; et
- afficher les données d'image médicale de guidage en temps réel sur l'interface utilisateur en temps réel.

9. Instrument médical selon la revendication 8, dans lequel le système d'imagerie médicale de guidage est l'un quelconque des suivants : un système de tomodensitométrie, un système d'imagerie par ultrasons, un système d'imagerie par résonance magnétique et un fluoroscope à rayons X.

10. Instrument médical selon l'une quelconque des revendications précédentes, dans lequel l'exécution des instructions exécutables par machine amène le système informatique à recevoir une image par résonance magnétique de planification (534) descriptive d'une région d'intérêt d'un sujet (408), dans lequel la segmentation anatomique identifie un emplacement de la structure anatomique dans l'image par résonance magnétique de planification, dans lequel le premier panneau est en outre configuré pour restituer une vue en coupe transversale de l'image par résonance magnétique de planification.

11. Instrument médical selon la revendication 10, dans lequel la mémoire stocke en outre un algorithme de segmentation automatisée (536) configuré pour générer la segmentation anatomique et/ou la segmentation de zone cible en réponse à l'entrée de l'image par résonance magnétique de planification, dans lequel l'exécution des instructions exécutables par machine amène en outre le processeur à générer la segmentation anatomique et/ou la segmentation de zone cible en entrant l'image par résonance magnétique de planification dans l'algorithme de segmentation automatisée.

12. Instrument médical selon la revendication 10 ou 11, dans lequel l'instrument médical comprend en outre un système d'imagerie par résonance magnétique de planification (502) configuré pour acquérir des données d'espace k de planification (532) descriptives du sujet, dans lequel la mémoire comprend en outre des commandes de séquence d'impulsions de planification (530) configurées pour commander le système d'imagerie par résonance magnétique afin d'acquérir les données d'espace k de planification, dans lequel l'exécution des instructions exécutables par machine amène en outre le système informatique à :

- commander le système d'imagerie par résonance magnétique de planification avec les commandes de séquence d'impulsions de planification afin d'acquérir les données d'espace k de planification ; et
- reconstruire l'image par résonance magnétique de planification à partir des données d'espace k de planification.

13. Instrument médical selon l'une quelconque des revendications précédentes, dans lequel l'affichage est un affichage tridimensionnel, dans lequel l'exécution des instructions exécutables par machine amène en outre le processeur à restituer le premier modèle tridimensionnel et le second modèle tridimensionnel de manière tridimensionnelle à l'aide de l'affichage tridimensionnel.

14. Programme informatique comprenant des instructions exécutables par machine (120) destinées à être exécutées par un système informatique (106) commandant un instrument médical (100, 200, 400, 500), dans lequel l'instrument médical comprend une interface utilisateur (108) comprenant un affichage ; dans lequel l'exécution des instructions exécutables par machine amène le système informatique à :

- recevoir (300) une segmentation anatomique (122) identifiant un emplacement d'une structure anatomique (416) ;
- recevoir (302) une segmentation de zone cible (124) identifiant un emplacement d'un volume (418) au moins partiellement à l'intérieur de la segmentation anatomique ; et
- afficher (304) une interface utilisateur graphique de planification (112) à l'aide de l'affichage ; dans lequel l'interface utilisateur graphique de planification comprend un premier panneau configuré pour restituer une vue en coupe transversale de la segmentation anatomique (136) et une vue en coupe transversale de la segmentation de zone cible (138) ; dans lequel l'interface utilisateur graphique de planification comprend en outre un deuxième panneau (132) configuré pour afficher un rendu d'un premier modèle tridimensionnel (140) de la segmentation anatomique et de la segmentation de zone cible ; dans lequel l'interface utilisateur graphique de planification comprend en outre un troisième panneau (134) configuré pour restituer un second modèle tridimensionnel (142) d'une partie restante de la segmentation de zone cible ; dans lequel l'interface utilisateur graphique de planification comprend en outre un sélecteur d'ablation (144, 144', 146) configuré pour fournir une zone d'ablation sélectionnable par l'utilisateur, descriptive d'un volume au moins partiellement à l'intérieur de la partie restante que l'utilisateur peut sélectionner pour ablater la segmentation de zone cible ;

dans lequel l'exécution des instructions exécutables par machine amène en outre le système informatique à, de manière itérative :

- recevoir (306) une zone d'ablation à partir du sélecteur d'ablation (144, 144', 146) ; et
- mettre à jour (308) la partie restante en supprimant la zone d'ablation de la partie restante, jusqu'à ce que les itérations correspondantes soient terminées.

15. Procédé non thérapeutique et non chirurgical de fonctionnement d'un instrument médical (100, 200, 400, 500), dans lequel l'instrument médical comprend une interface utilisateur (108), dans lequel l'interface utilisateur comprend un affichage,

dans lequel le procédé comprend :

- la réception (300) d'une segmentation anatomique (122) identifiant un emplacement d'une structure anatomique (416) ;
- la réception (302) d'une segmentation de zone cible (124) identifiant un emplacement d'un volume (416) au moins partiellement à l'intérieur de la segmentation anatomique ; et
- l'affichage (304) d'une interface utilisateur graphique de planification (112) à l'aide de l'affichage ; dans lequel l'interface utilisateur graphique de planification comprend un premier panneau (130) configuré pour restituer une vue en coupe transversale de la segmentation anatomique (136) et de la segmentation de zone cible (138) ; dans lequel l'interface utilisateur graphique de planification comprend en outre un deuxième panneau (132) configuré pour afficher un rendu d'un premier modèle tridimensionnel (140) de la segmentation anatomique et de la segmentation de zone cible ; dans lequel l'interface utilisateur graphique de planification comprend en outre un troisième panneau (134) configuré pour restituer un second modèle tridimensionnel (142) d'une partie restante de la segmentation de zone cible ; dans lequel l'interface utilisateur graphique de planification comprend en outre un sélecteur d'ablation (144, 144', 146) configuré pour fournir une zone d'ablation sélectionnable par l'utilisateur, descriptive d'un volume au moins partiellement à l'intérieur de la partie restante que l'utilisateur peut sélectionner pour ablater la segmentation de zone cible ;

dans lequel le procédé comprend de manière itérative :

- la réception (306) d'une zone d'ablation à partir du sélecteur d'ablation (144, 144', 146) ; et
- la mise à jour (308) de la partie restante en supprimant la zone d'ablation de la partie restante, jusqu'à ce que les itérations correspondantes soient terminées.

Fig. 1

Fig. 2

receive anatomical
segmentation ⟶ 300

receive target zone
segmentation ⟶ 302

display planning
GUI ⟶ 304

receive ablation
zone ⟶ 306

update remaining
portion ⟶ 308

312

No ⟵ iterations
finished? — Yes ⟶ end

310

Fig. 3

Fig. 4

Fig. 5

Fig. 6

Fig. 7

Ablation coverage
LESION 1
Planned 88%
Ablated 0%

144

142          134

Fig. 8

Fig. 9

Fig. 10

**EP 4 135 613 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150320509 A1